# EUROPEAN PATENT SPECIFICATION

(11) **EP 4 065 339 B1**
(45) Date of publication and mention of the grant of the patent: **16.07.2025**
(21) Application number: 20893969.4
(22) Date of filing: 18.11.2020
(51) Int. Cl.: B29D 23/18, B29C 43/22, B29C 55/30, B29C 53/12, B29C 53/14, B29C 53/22, D02J 1/02, D02G 1/02, D01D 5/20, D01D 5/24, D01D 5/34, D01D 10/00, A61F 2/08

(54) **METHODS OF FORMING A FIBER, FIBER ACTUATORS, FIBER BUNDLE AND METHOD OF FORMING THE FIBER BUNDLE**
VERFAHREN ZUR HERSTELLUNG EINER FASER, FASERAKTUATOREN, FASERBÜNDEL UND VERFAHREN ZUR HERSTELLUNG DES FASERBÜNDELS
PROCÉDÉS DE FORMATION D'UNE FIBRE, ACTIONNEURS À FIBRES, FAISCEAU DE FIBRES ET PROCÉDÉ DE FORMATION DU FAISCEAU DE FIBRES

(30) Priority: 25.11.2019 SG 10201911087V
(43) Date of publication of application: 05.10.2022
(73) Proprietor: Nanyang Technological University, Singapore 639798 (SG)
(72) Inventor: WEI, Lei, Singapore 639798 (SG); WANG, Zhixun, Singapore 639798 (SG); CHEN, Mengxiao, Singapore 639798 (SG)
(74) Representative: Schiweck Weinzierl Koch Patentanwälte Partnerschaft mbB
(86) International application number: PCT/SG2020/050668
(87) International publication number: WO 2021/107865

(56) References cited:
- EP-A1- 0 122 828
- KR-A- 20080 091 816
- US-A- 4 028 081
- US-A- 4 389 364
- US-A- 4 473 273
- US-A1- 2009 032 991
- US-A1- 2017 054 069
- US-A1- 2019 233 972
- US-B1- 8 641 732
- KANIK MEHMET, ORGUC SIRMA, VARNAVIDES GEORGIOS, KIM JINWOO, BENAVIDES THOMAS, GONZALEZ DANI, AKINTILO TIMOTHY, TASAN C CEM, CHANDR: "Strain-Programmable Fiber-Based Artificial Muscle", SCIENCE, vol. 365, no. 6449, 12 July 2019 (2019-07-12), pages 145 - 150, XP055831155, DOI: 10.1126/ SCIENCE .AAW2502

## Description

### Technical Field

Various embodiments relate to methods of forming a fiber, fiber actuators, a fiber bundle and a method of forming the fiber bundle.

### Background

A bundle of muscle fibers forms a muscle of actual creatures. This structure is favoured by nature and has its advantages, such as high flexibility, and the ability of maintaining its actuation even when some fibers are broken. Artificial muscle using fibers and fiber bundle structures possess the same advantages. However, current methods for fiber actuators have limited capacity on scalable fabrication and the integration of actuators and sensors.

KR20080091816A discloses non-fractured, non-fibrillatable short fibers, for reinforcing matrix materials such as concrete, have substantially uniform transverse cross-sectional areas along their length for maximum efficiency in pull-out resistance, and two different tapering characteristics along their lengths. Preferred bi-tapered fibers of the invention have a high modulus of elasticity in the range of 5-250 Gigapascal and are preferably modulated in both tapering dimensions. Matrix materials containing the fibers, as well as a method for making the fibers, are disclosed in this document. US 8641732B1 discloses a self-retaining suture comprises a variable-dimension filament which varies in size and/or shape along the length of the filament. The variation in size and/or shape results in a variation in the distribution of filament material from one region of the filament to the next. The filament has retainers formed in the surface such that the filament can engage and retain tissue. The retainers are formed in a manner that makes use of regions of the filament where additional material is distributed. The resulting self-retaining suture has a greater minimum cross-section than would be created using an equivalent uniform filament. The resulting self-retaining suture consequently has a greater tensile strength. Methods for manufacturing the filament and retainers are also described in this document.

There is, therefore, need for techniques that seek to address the above-mentioned problems associated with the prior art.

### Summary

The invention is defined in the independent claims. Further embodiments of the invention are defined in the dependent claims.

According to an embodiment, a method of forming a fiber is provided. The method may include subjecting a preform to a thermal drawing process to draw the preform into a fiber, and applying, at intervals, a compressive force to sections of the fiber along a length of the fiber during the thermal drawing process to reduce a dimension of the sections to form the fiber into an accordion-like fiber.

According to an embodiment, a method of forming a fiber is provided. The method may include subjecting a preform to a thermal drawing process to draw the preform into a fiber, and inducing a relative rotational movement between the preform and a region of the fiber during the thermal drawing process to twist the fiber until the twisted fiber becomes coiled to form a spring-like fiber.

According to an embodiment, a fiber actuator having the accordion-like fiber formed by the method as described herein is provided.

According to an embodiment, a fiber actuator having the spring-like fiber formed by the method as described herein is provided.

According to an embodiment, a fiber bundle is provided. The fiber bundle may include at least one fiber actuator as described herein, and at least one functional fiber configured to function as at least one of a sensor or an energy harvester.

According to an embodiment, a method of forming a fiber bundle is provided. The method may include arranging at least one fiber actuator as described herein and at least one functional fiber configured to function as at least one of a sensor or an energy harvester to form the fiber bundle.

### Brief Description of the Drawings

In the drawings, like reference characters generally refer to like parts throughout the different views. The drawings are not necessarily to scale, emphasis instead generally being placed upon illustrating the principles of the invention. In the following description, various embodiments of the invention are described with reference to the following drawings, in which:
FIG. 1A shows a flow chart illustrating a method of forming a fiber, according to various embodiments.
FIG. 1B shows a flow chart illustrating a method of forming a fiber, according to various embodiments.
FIG. 1C shows a schematic view of a fiber bundle, according to various embodiments.
FIG. 1D shows a method of forming a fiber bundle, according to various embodiments.
FIG. 2A shows a schematic view illustrating fabrication of an accordion-like fiber structure, according to various embodiments.
FIGS. 2B and 2C show, in schematic cross-sectional and top views, the steps for fabricating an accordion-like fiber when the iris is respectively expanded and contracted to compress the fiber.
FIG. 3 shows a schematic view illustrating fabrication of a twisted or spring-like fiber structure, according to various embodiments.
FIGS. 4A and 4B show schematic cross-sectional views of fiber sensors, according to various embodiments.
FIGS. 5A to 5C show schematic cross-sectional views of fiber-shaped lateral pressure sensors, according to various embodiments.
FIGS. 6A to 6C show schematic cross-sectional views illustrating integration of multifunctional fiber bundles using fiber-shaped actuators and sensors, according to various embodiments. FIG. 6D shows a schematic cross-sectional view along line A-A' shown in FIG. 6A.
FIGS. 7A to 7D show schematic diagrams illustrating operation of an energy harvesting fiber of various embodiments.

### Detailed Description

The following detailed description refers to the accompanying drawings that show, by way of illustration, specific details and embodiments in which the invention may be practiced. These embodiments are described in sufficient detail to enable those skilled in the art to practice the invention. Other embodiments may be utilized and structural, logical, and electrical changes may be made without departing from the scope of the invention. The various embodiments are not necessarily mutually exclusive, as some embodiments can be combined with one or more other embodiments to form new embodiments.

Embodiments described in the context of one of the methods or devices are analogously valid for the other methods or devices. Similarly, embodiments described in the context of a method are analogously valid for a device, and vice versa.

Features that are described in the context of an embodiment may correspondingly be applicable to the same or similar features in the other embodiments. Features that are described in the context of an embodiment may correspondingly be applicable to the other embodiments, even if not explicitly described in these other embodiments. Furthermore, additions and/or combinations and/or alternatives as described for a feature in the context of an embodiment may correspondingly be applicable to the same or similar feature in the other embodiments.

In the context of various embodiments, the articles "a", "an" and "the" as used with regard to a feature or element include a reference to one or more of the features or elements.

In the context of various embodiments, the term "about" as applied to a numeric value encompasses the exact value and a reasonable variance.

As used herein, the term "and/or" includes any and all combinations of one or more of the associated listed items.

As used herein, the phrase of the form of "at least one of A or B" may include A or B or both A and B. Correspondingly, the phrase of the form of "at least one of A or B or C", or including further listed items, may include any and all combinations of one or more of the associated listed items.

Various embodiments may provide methods for or of forming 3D (three-dimensional)-shaped fiber structures and one or more fiber bundle assemblies with multiple functionalities.

The techniques disclosed herein may enable or use preform-to-fiber fabrication to achieve 3D-shaped fiber structures, for example, accordion-like fiber structures, and twisted or spring-like fiber structures along the (entire) length of the fibers, for example, for actuation and/or sensing.

Various embodiments may provide one or more fiber-shaped actuators and sensors and employing the actuator(s) and sensor(s) to form one or more fiber bundles may achieve the integration of actuation and sensing. Further, the thermal drawing methods of various embodiments may ensure readily available fabrication in a large scale manner.

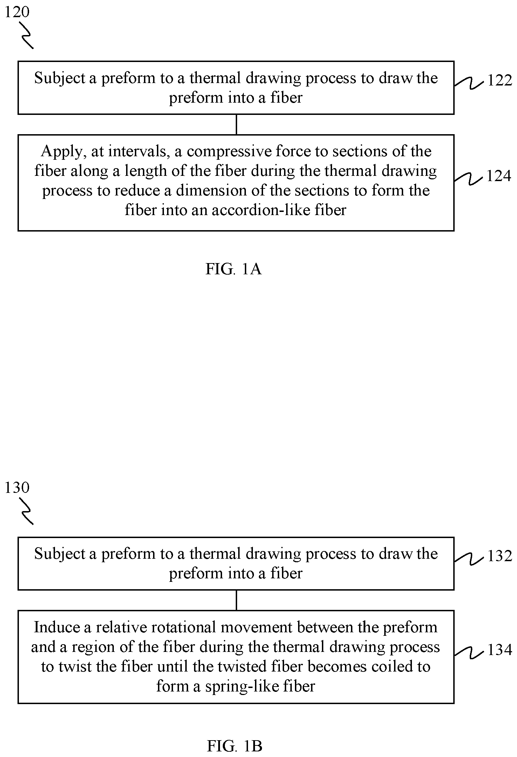

FIG. 1A shows a flow chart 120 illustrating a method of forming a fiber, according to various embodiments.

At 122, a preform is subjected to a thermal drawing process to draw the preform into a fiber. This may mean that a fiber preform is heated to a sufficiently high temperature in a thermal drawing process for it to be drawn into a fiber. As non-limiting examples, the drawing temperature may be in the range of between about 100°C and about 500°C, depending on the material(s) used in the preform, e.g., between about 100°C and about 400°C, between about 100°C and about 300°C, between about 200°C and about 500°C, or between about 200°C and about 300°C. The drawing temperature may be chosen so as to allow a viscosity of the preform material(s) to be around 10⁵ Pa s. Nevertheless, it should be appreciated that in some embodiments, the drawing temperature may be lower than 100°C or higher than 500°C.

In various embodiments, the fiber drawing rate or drawing speed may be in the range of between about 0.1 m/min and about 10 m/min, depending on the draw-down ratio (i.e., preform-to-fiber ratio), e.g., between about 0.1 m/min and about 5 m/min, between about 0.1 m/min and about 2 m/min, between about 0.1 m/min and about 1 m/min, between about 1 m/min and about 8 m/min, between about 1 m/min and about 5 m/min, between about 3 m/min and about 8 m/min, or between about 5 m/min and about 10 m/min.

At 124, a compressive force is applied, at intervals, to sections of the fiber along a length of the fiber during the thermal drawing process to reduce a dimension of the sections to form the fiber into an accordion-like fiber. This means that the compressive force is applied to the sections of the fiber such that the sections are of a reduced (cross-sectional) dimension to form the fiber into an accordion-like fiber. The compressive force may be applied to an external surface or perimeter or circumference of the fiber. The reduced dimension may mean a reduced perimeter or circumference. This means that the sections of the fiber where the compressive force is applied are narrower sections compared to parts of the fiber which have not been subjected to the compressive force. A mechanical force may be applied, at the intervals, to the sections of the fiber to apply the compressive force.

In the context of various embodiments, the compressive force applied to the sections of the fiber may be at a level of lower than 1 N or a few newtons. As non-limiting examples, the compressive force may be in the range of between about 0.5 N and about 10 N, for example, between about 0.5 N and about 5 N, between about 0.5 N and about 3 N, between about 0.5 N and about 1 N, between about 1 N and about 10 N, between about 3 N and about 10 N, between about 5 N and about 10 N, between about 3 N and about 8 N, or between about 3 N and about 5 N.

In various embodiments, the compressive force may be applied to reduce or narrow the sections to about 50% of the (original) diameter of the (accordion-like) fiber. For example, the fiber diameter may be in the range of between about 0.1 mm and about 1 mm, and the sections may be correspondingly reduced to between about 0.05 mm and about 0.5 mm. Nevertheless, it should be appreciated that the reduction may be adjustable and not limited to 50%. As non-limiting examples, the compressive force may be applied to the sections of the fiber to reduce or narrow the sections to between about 30% and about 70% of the diameter of the (accordion-like) fiber, for example, between about 30% and about 50%, between about 30% and about 40%, between about 50% and about 70%, between about 40% and about 60%, or between about 45% and about 55%.

In various embodiments, the preform has a hollow core defined therein, i.e., a hollow core preform.

In various embodiments, at 124, the fiber may be passed through an iris, and the iris may be closed, at the intervals, to apply the compressive force to the sections of the fiber. The iris may be opened and closed continually such that the iris is operable to close at the intervals to apply the compressive force.

In various embodiments, rather than setting the amount of compressive force, the state or position of the iris, (i.e., original/open position and contracted/closed position) may be controlled to apply the required amount of compressive force, for example, by controlling how much the iris contracts or how much the fiber is compressed by the iris.

The time interval, t1, between applications of two successive compressive forces (i.e., how much time passes between applying a compressive force and applying the next compressive force), and/or the compression time, t2, (i.e., how long the compressive force is applied each time), may determine the ratio and/or density of the sections (of reduced dimension) of the accordion-like fiber.

As a non-limiting example, to fabricate a fiber with 50% narrow sections (i.e., an accordion-like fiber having narrow sections, where the compressive force has been applied to, occupying about 50% of the length of the accordion-like fiber), at a drawing speed, V, and employing an iris having a thickness, W (corresponding to the width of the sections the compressive force is applied to), the relationship between the time interval, t1, and the compression time, t2, may be defined by W+t2*V=t1*V (how far the narrow sections are apart). The compression time, t2, may be adjusted for controlling how many narrow sections are to be formed. To reach a higher density of the narrow sections (i.e., higher number of narrow sections), t2, may be adjusted to be as short as required.

Using the non-limiting example of a drawing speed, V, of about 1 m/min, a thickness, W, of the iris of about 0.5 mm, a compression time, t2, of about 0.5s, and a time interval, t1, of about 0.53 s, the narrow sections that may be formed may take up about 50% of the fiber and the distance between adjacent sections (of reduced dimension) may be about 8.85 mm. Adjusting the compression time, t2, to about 0. 1s, and the time interval, t1, to about 1.3 s, the ratio of the narrow sections to the fiber may be maintained the same at 50%, although the distance between adjacent sections (of reduced dimension) may be reduced to about 2.17 mm.

It should be appreciated that the various process parameters, including the time interval, t1, the compression time, t2, and the thickness, W, of the iris (corresponding to the width of the sections the compressive force is applied to), may be adjusted for larger or smaller density of the narrow sections relative to the fiber.

In various embodiments, the thickness, W, of the iris may be between about 0.05 mm and about 0.5 mm, for example, between about 0.05 mm and about 0.3 mm, between about 0.05 mm and about 0.2 mm, between about 0.05 mm and about 0.1 mm, between about 0.1 mm and about 0.5 mm, between about 0.3 mm and about 0.5 mm, or between about 0.1 mm and about 0.3 mm.

In the context of various embodiments, the compressive force may be applied at time intervals (i.e., the time intervals, t1, between two successive compressive forces) of between about 0.001s and about 3s, for example, between about 0.001s and about 1s, between about 0.001s and about 0.5s, between about 0.001s and about 0.1s, between about 0.001s and about 0.05s, between about 0.001s and about 0.01s, between about 0.01s and about 3s, between about 0.1s and about 3s, between about 1s and about 3s, between about 0.05s and about 1s, or between about 0.1s and about 1s.

In the context of various embodiments, the compressive force may be applied, for each of the sections of the fiber, for between about 0.001s and about 3s. This corresponds to the compression time, t2, corresponding to each application of compressive force. As non-limiting examples, the compressive force may be applied, for each of the sections of the fiber, for between about 0.001s and about 1s, between about 0.001s and about 0.5s, between about 0.001s and about 0.1s, between about 0.001s and about 0.05s, between about 0.001s and about 0.01s, between about 0.01s and about 3s, between about 0.1s and about 3s, between about 1s and about 3s, between about 0.05s and about 1s, or between about 0.1s and about 1s.

In the context of various embodiments, a distance or spacing between (two) adjacent sections of the sections of the fiber may be between about 0.05 mm and about 10 mm, for example, between about 0.05 mm and about 5 mm, between about 0.05 mm and about 3 mm, between about 0.05 mm and about 1 mm, between about 0.1 mm and about 10 mm, between about 1 mm and about 10 mm, between about 5 mm and about 10 mm, between about 0.1 mm and about 5 mm, between about 1 mm and about 5 mm, between about 1 mm and about 3 mm.

In the context of various embodiments, a density or proportion or percentage of the sections of the fiber relative to the (accordion-like) fiber (as a whole) may be between about 10% and about 80%, for example, between about 10% and about 50%, between about 10% and about 30%, between about 50% and about 80%, between about 30% and about 80%, between about 30% and about 50%, between about 20% and about 60%, or between about 40% and about 60%.

In various embodiments, the preform may include a thermoplastic. The thermoplastic may include a material that may be responsive to at least one of heat, electric field, light (or optical signal), chemical or vapour. For example, the volume of the material may change in response to one or more of heat, electric field, light, chemical or vapour.

Various embodiments may further provide a fiber actuator having the accordion-like fiber formed by the method as described in the context of the flow chart 120.

FIG. 1B shows a flow chart 130 illustrating a method of forming a fiber, according to various embodiments.

At 132, a preform is subjected to a thermal drawing process to draw the preform into a fiber. This may mean that a fiber preform is heated to a sufficiently high temperature in a thermal drawing process for it to be drawn into a fiber. As non-limiting examples, the drawing temperature may be in the range of between about 100°C and about 500°C, depending on the material(s) used in the preform, e.g., between about 100°C and about 400°C, between about 100°C and about 300°C, between about 200°C and about 500°C, or between about 200°C and about 300°C. The drawing temperature may be chosen so as to allow a viscosity of the preform material(s) to be around 10⁵ Pa s. Nevertheless, it should be appreciated that in some embodiments, the drawing temperature may be lower than 100°C or higher than 500°C.

In various embodiments, the fiber drawing rate or drawing speed may be in the range of between about 0.1 m/min and about 10 m/min, depending on the draw-down ratio (i.e., preform-to-fiber ratio), e.g., between about 0.1 m/min and about 5 m/min, between about 0.1 m/min and about 2 m/min, between about 0.1 m/min and about 1 m/min, between about 1 m/min and about 8 m/min, between about 1 m/min and about 5 m/min, between about 3 m/min and about 8 m/min, or between about 5 m/min and about 10 m/min.

At 134, a relative rotational movement is induced between the preform and a region of the fiber during the thermal drawing process to twist the fiber until the twisted fiber becomes coiled to form a spring-like fiber. In various embodiments, one of the preform and the region of the fiber may be rotated relative to the other of the preform and the region of the fiber during the thermal drawing process to twist the fiber. The other of the preform and the region of the fiber may be fixed in place. In further embodiments, both the preform and the region of the fiber may be rotated relative to one another. As may be appreciated, as twisting of the fiber progresses, more twist is incorporated into the fiber, and this leads to the twisted fiber becoming coiled. The region of the fiber may include a section or any section of the fiber, including, for example, an end region of the fiber opposite to the preform.

In the context of various embodiments, the rotation rate of the preform and/or the region of the fiber may be between about 50 rpm and about 20000 rpm, for example, between about 50 rpm and about 10000 rpm, between about 50 rpm and about 5000 rpm, between about 50 rpm and about 1000 rpm, between about 50 rpm and about 500 rpm, between about 1000 rpm and about 20000 rpm, between about 5000 rpm and about 20000 rpm, between about 10000 rpm and about 20000 rpm, between about 1000 rpm and about 10000 rpm, between about 1000 rpm and about 5000 rpm, or between about 10000 rpm and about 20000 rpm.

In various embodiments, at 134, the preform and the region of the fiber may be rotated in opposite directions. In other words, both the preform and the end of the fiber may be rotated to twist the fiber. Rotation of both the preform and the end of the fiber may be carried out simultaneously.

In the context of various embodiments, a diameter of each coil and/or a diameter of the spring-like fiber may be in the range from hundreds of micrometer to a few millimeters, for example, between about 100 µm and about 10 mm (10000 µm), e.g., between about 100 µm and about 5 mm, between about 100 µm and about 1 mm, between about 100 µm and about 500 µm, between about 500 µm and about 1 mm, between about 500 µm and about 5 mm, between about 1 mm and about 10 mm, or between about 1 mm and about 5 mm.

In the context of various embodiments, a (twist) period between adjacent coils of the spring-like fiber (i.e., distance between adjacent coils) may be in the range from hundreds of micrometer to a few millimeters, for example, between about 100 µm and about 10 mm (10000 µm), e.g., between about 100 µm and about 5 mm, between about 100 µm and about 1 mm, between about 100 µm and about 500 µm, between about 500 µm and about 1 mm, between about 500 µm and about 5 mm, between about 1 mm and about 10 mm, or between about 1 mm and about 5 mm.

In the context of various embodiments, a number of coils of the spring-like fiber, for each meter of the spring-like fiber, may be between 100 and 10000. The number of coils that may be formed in the spring-like fiber may be determined by dividing the twist period with the length of the spring-like fiber. Long lengths of fibers (e.g., 100s of meters) may be readily fabricated. Considering a length of 1 meter of the spring-like fiber or for each length of 1 meter of the spring-like fiber, the number of coils may be in the range of a few hundreds to a few thousands, for example, between 100 and 10000, e.g., between 100 and 5000, between 100 and 1000, between 100 and 500, between 500 and 10000, between 1000 and 10000, between 5000 and 10000, between 500 and 5000, or between 1000 and 5000.

In various embodiments, the preform may include or have a solid core.

In various embodiments, the preform may include a thermoplastic. The thermoplastic may include a material that may be responsive to at least one of heat, electric field, light (or optical signal), chemical or vapour. For example, the volume of the material may change in response to one or more of heat, electric field, light, chemical or vapour.

Various embodiments may further provide a fiber actuator having the spring-like fiber formed by the method as described in the context of the flow chart 130.

In the context of various embodiments, the material responsive to heat may include at least one of ethylene-vinyl acetate, (high-density) polyethylene, or poly (ε-caprolactone) (PCL).

In the context of various embodiments, the material responsive to electric field may include polyacrylonitrile or polyurethane.

In the context of various embodiments, the material responsive to light may include liquid crystal network coated polyethylene terephthalate (PET).

In the context of various embodiments, the material responsive to chemical or vapour may include poly (ε-caprolactone) with nanofillers (e.g., carbon nanotubes).

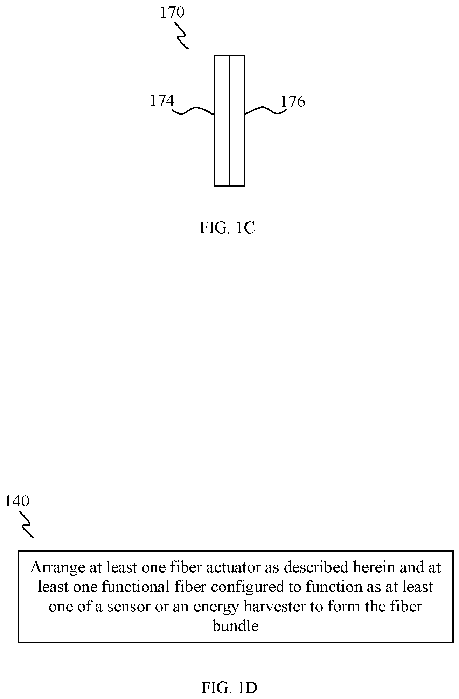

FIG. 1C shows a schematic view of a fiber bundle 170, according to various embodiments. The fiber bundle 170 includes at least one fiber actuator (represented schematically by rectangle 174) as described herein, and at least one functional fiber (represented schematically by rectangle 176) configured to function as at least one of a sensor or an energy harvester. This means that there may be provided at least one fiber 176 capable of providing a sensing function and/or an energy harvesting function. In other words, the at least one fiber 176 may be a sensing fiber and/or an energy harvesting fiber. Each fiber actuator 174 may be the accordion-like fiber or the spring-like fiber described herein. The accordion-like fiber may be formed using the method described in the context of the flow chart 120. The spring-like fiber may be formed using the method described in the context of the flow chart 130.

In various embodiments, a plurality of fiber actuators 174 may be provided, where a respective fiber actuator may be the accordion-like fiber or the spring-like fiber described herein.

In various embodiments, a plurality of functional fibers 176 may be provided, where a respective functional fiber may be configured to function as a sensor and/or an energy harvester.

In various embodiments, the at least one functional fiber 176 may include a shell having a thermoplastic polymer. The shell may be of any suitable shapes.

In various embodiments, the at least one functional fiber 176 may include a core having a thermoelectric glass or a conductive material (e.g., to act as an electrode). The core may be of any suitable shapes. The at least one functional fiber 176 having the thermoelectric glass in the core may be configured as a thermal or temperature sensor. The at least one functional fiber 176 having the conductive material in the core may be configured as a distance and/or force sensor. Additionally or alternatively, the at least one functional fiber 176 having the conductive material in the core may be configured as an energy harvesting fiber.

In various embodiments, the at least one functional fiber 176 may include a pair of electrodes arranged spaced apart.

In various embodiments, the at least one functional fiber 176 may further include an insulative core (e.g., a core having an electrically insulative material), wherein the pair of electrodes may be arranged on opposite sides of the insulative core. The pair of electrodes may be in contact with the insulative core. The insulative core may be deformable.

In various embodiments, the at least one functional fiber 176 may include a deformable core, and a pair of electrodes arranged on opposite sides of the deformable core. The pair of electrodes may contact the deformable core. The deformable core may be of any suitable shapes. The deformable core may include air or liquid or polymer. Each electrode may be of any suitable shapes. Each electrode includes a conductive material.

The at least one functional fiber 176 having the deformable core and the pair of electrodes may be configured as a lateral pressure sensor. The pair of electrodes may define a plane-parallel capacitor. If a pressure applied to the at least one functional fiber causes a change in the distance between the pair of electrodes, resulting from the deformation of the deformable core, the capacitance of the capacitor changes, thus allowing sensing of the (lateral) pressure.

The at least one functional fiber 176 having the deformable core and the pair of electrodes may, additionally or alternatively, be configured as an energy harvesting fiber.

FIG. 1D shows a method 140 of forming a fiber bundle, according to various embodiments. At least one fiber actuator as described herein and at least one functional fiber configured to function as at least one of a sensor or an energy harvester are arranged to form the fiber bundle.

For forming the fiber bundle, the method may include forming the at least one fiber actuator according to any one of the methods described herein, forming the at least one functional fiber, defining a hollow spacing in one of the at least one fiber actuator and the at least one functional fiber, and inserting the other of the at least one fiber actuator and the at least one functional fiber into the hollow spacing. This may mean that a hollow spacing or hollow channel may be defined in the at least one fiber actuator or the at least one functional fiber, and the fiber with the hollow spacing is configured to receive, in the hollow spacing, the other fiber.

The at least one fiber actuator may be the accordion-like fiber or the spring-like fiber described herein. The accordion-like fiber may be formed using the method described in the context of the flow chart 120. The spring-like fiber may be formed using the method described in the context of the flow chart 130.

**In** various embodiments, the at least one fiber actuator may be fabricated first, followed by the fabrication of the at least one functional fiber.

**In** further embodiments, the at least one functional fiber may be fabricated first, followed by the fabrication of the at least one fiber actuator.

While the methods described above is illustrated and described as a series of steps or events, it will be appreciated that any ordering of such steps or events are not to be interpreted in a limiting sense. For example, some steps may occur in different orders and/or concurrently with other steps or events apart from those illustrated and/or described herein. In addition, not all illustrated steps may be required to implement one or more aspects or embodiments described herein. Also, one or more of the steps depicted herein may be carried out in one or more separate acts and/or phases.

Various embodiments may provide a method of fabricating a 3D-shaped fiber including thermal drawing a fiber from a hollow core fiber preform, and subjecting the fiber to a compression force such that the fiber forms a 3D-shaped fiber (e.g., an accordion-like fiber).

Various embodiments may provide a method of fabricating a 3D-shaped fiber including rotating a fiber preform during thermal drawing process such that the drawn fiber is spiral-shaped (e.g., a spiral-shaped fiber or a spring-like fiber).

Various embodiments may provide a fiber arrangement (or fiber bundle) having at least one 3D-shaped fiber (e.g., a spiral-shaped fiber), and at least one fiber having a sensing and/or energy harvesting function.

Various embodiments may provide a fiber bundle having a plurality of hollow cores, wherein one or more hollow cores are provided with materials configured to perform a sensing and/or energy harvesting function.

In various embodiments, the sensing function may include at least one of temperature, distance, force or pressure sensing. The fiber having a sensing function may be formed by a thermal drawing process.

For a fiber having a temperature sensing function, the fiber may have a core made of thermoelectric glass and a cladding made of thermoplastic polymer. For a fiber having distance and/or force sensing function, the fiber may have a core made of conductive electrode material and a cladding made of thermoplastic polymer. For a fiber having pressure sensing function, the fiber may have a cladding, a core and two electrodes arranged adjacent to the core, where the electrodes may include metal or conductive polymer, the core may include air, liquid or polymer, and the cladding may include thermoplastic polymer.

For a fiber for energy harvesting, the fiber may have a cladding, a core and two electrodes arranged adjacent to the core. The electrodes may include conductive materials, metal, liquid metal or conductive polymer, the core may include air, liquid or polymer, and the cladding may include thermoplastic polymer.

Various embodiments will now be further described by way of the following non-limiting examples and with reference to the figures.

Various embodiments may provide methods for forming 3D-shaped fiber actuators (or 3D-shaped fiber actuation structures). The methods may be employed to fabricate fibers having an accordion-like structure, and fibers having a twisted or spring-like structure.

In various embodiments for forming accordion-like fibers (or fiber structures), a macro preform, for example, a hollow-core perform (which may be cylindrical), may be prepared with a thermoplastic whose volume may change in response to or under one or more certain stimuli. Non limiting examples of the stimuli may include thermal, electrical field, chemical concentration, light waves, etc.

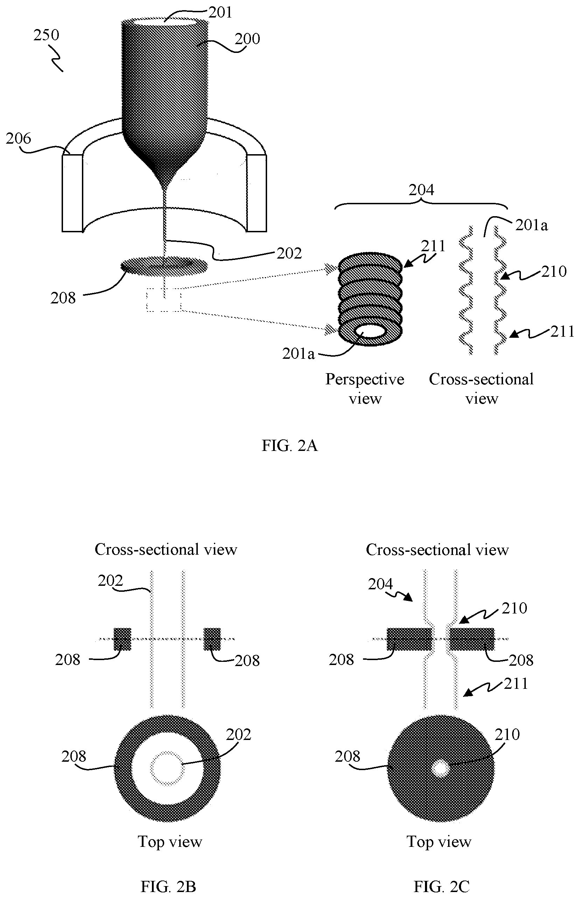

FIG. 2A shows a schematic view illustrating fabrication of an accordion-like fiber 204. The accordion-like fiber 204 may be fabricated during a thermal fiber drawing process. The set-up 250 for fabricating the accordion-like fiber 204 may include a furnace or heater 206 and an iris 208. During the fiber drawing process, a suitable preform 200 with a hollow core 201 may be fed to the furnace 206 where the preform 200 is heated above its glass transition temperature till soft. The softened preform 200 may then flow and form (or drawn) into a fiber 202, which is generally a scaled down version of the preform 200. Through a hot-pressing process, the resulting fiber 202 may be compressed or subjected to a compressive force after leaving the furnace 206, with the fiber 202 still being in the viscous state. The compressive force may be applied to sections of the fiber 202 at a certain time interval to form the accordion-like structure 204. The compressive force may be mechanical force. Sections of the fiber 202 may be subjected to a compressive force from the outside of the fiber 202 applied to the surface of the fiber 202. The compressive force may be applied to the sides of the fiber 202 or around or along the perimeter or circumference of the fiber 202. The accordion-like fiber 204 that may be fabricated is shown, in FIG. 2A, in a perspective view and a cross-sectional view along a longitudinal section of the fiber 204. Such a structure may allow the resulting fiber 204 to store some length in the wrinkles, and may provide tensile stroke when the volume of the fiber 204 changes (e.g., expands) in response to or under one or more certain stimuli. The change in the volume of the fiber 204 may effectively be due to a volume change of the material the fiber 204 is made of, which may be a thermoplastic.

Referring to FIGS. 2A to 2C, as a non-limiting example, an iris 208 may be used to apply the compressive force. The iris 208 may be made of a rigid material and having the functions of being contracted (or closed) and expanded (or opened). The iris 208 may be arranged surrounding the fiber 202 so that the fiber passes through the iris 208. As illustrated in FIG. 2B, when the iris 208 is in the open or expanded state, the drawn straight fiber 202 may pass through the iris 208. As illustrated in FIG. 2C, when the iris 208 is in the closed or contracted state, the iris 208 compresses a section of the fiber 202 (which remains at a sufficiently high temperature and having a viscosity) that the iris 208 comes into contact with, thereby forming or defining a narrower section or part 210 on the fiber 202, compared to the uncompressed section 211 of the fiber 202 having the initial diameter or cross-sectional dimension of the drawn fiber 202. In other words, by pressing the iris 208 onto the fiber 202, fiber 202 is made narrower at certain positions to define narrower sections 210. Repeating the process, that is by expanding and contracting the iris 208, at a defined time interval (which may be regular or irregular) creates an accordion-like fiber 204. As the preform 200 has a hollow core 201, the accordion-like fiber 204 also has a hollow core 201a. The accordion-like fiber structure 204 may be a hollow-core straight fiber, regardless of its surface configuration or geometry.

In various embodiments for forming twisted or spring-like fibers (or fiber structures), the twist may be incorporated as-drawn and/or post-drawn. Twisted or spring-like fibers may provide both tensile and torsional strokes when the fiber material undergoes a volume change.

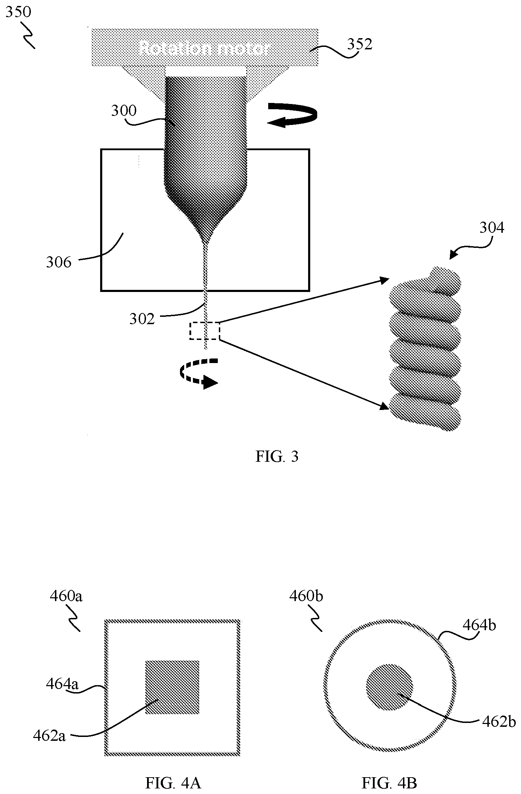

FIG. 3 shows a schematic view illustrating fabrication of a twisted or spring-like fiber 304. The twisted or spring-like fiber 304 may be fabricated during a thermal fiber drawing process and/or after the drawing process Twisting the preform and/or the fiber in or during a thermal drawing process may provide a robust method to form a coiled fiber structure, compared to post-processing, as it may offer larger scale fabrication and/or lower diffculty in manufacturing as the fiber has a (sufficient) viscosity after having exited or having been pulled out of the furnace. FIG. 3 shows, as a non-limiting example, a set-up 350 for fabricating the spring-like fiber 304 during the drawing process. The set-up 350 may include a furnace or heater 306 and a rotation motor 352. The rotation motor 352 may hold or clamp a suitable preform 300 that is to be fed to the furnace 306. The preform 300 may have a solid core. When a fiber 302 is drawn, the end of the fiber 302 may be fixed in place.

During the drawing process, the preform 300 is heated by the furnace 306 above its glass transition temperature till soft. At the same time, during thermal drawing, the rotation motor 352 supporting the preform 300 rotates (e.g., in a direction as represented by the solid curve arrow adjacent the preform 300 but the rotation is not limited to such direction), thereby rotating the preform 300. In other words, the preform 300 is subjected to a rotational force, resulting in a rotational movement. The softened preform 300 may then flow and form (or drawn) into a fiber 302, which is generally a scaled down version of the preform 300, where, with the end of the fiber 302 being a fixed end, the fiber 302 becomes twisted as a result of the preform 300 being rotated by the motor 352. As the process progresses, with more twist incorporation, the fiber 302 becomes more twisted or has a higher degree of twist and the twisted fiber 302 eventually becomes coiled, thereby forming a spring-like fiber structure 304. The spring-like fiber structure 304 with a twisted structure formed with coiling may be a thicker solid-core fiber compared to the accordion-like fiber 204 (FIG. 2A).

In other embodiments, instead of the rotation motor 352 clamping and rotating the preform 300, the fiber 302 may be twisted by connecting a rotation motor (e.g., similar to rotation motor 352) to a capstan that supports the drawn fiber 302. The rotation motor may rotate the capstan during the drawing process and/or after the drawing process (with the preform 300 being fixed), thereby rotating the fiber 302 (e.g., in a direction as represented by the dashed curve arrow adjacent the fiber 302 but the rotation is not limited to such direction) and twisting the fiber 302, and eventually forming the spring-like fiber structure 304. While it may be sufficient to rotate either the preform 300 or the end of the fiber 302, in further embodiments for forming the spring-like fiber structure 304, both the preform 300 and the end of the fiber 302 may be rotated, relative to each other, in opposite directions.

In various embodiments, each of the accordion-like fiber 204 and the spring-like fiber 304 may be used as an actuator (or actuator fiber), or as part of an actuation structure, for example, for applications in an artificial muscle system. The volume of each of the accordion-like fiber 204 and the spring-like fiber 304 may change in response to one or more stimuli. As a non-limiting example, each fiber 204, 304 may be coupled to two structures or elements, and, in response to the application of heat to the fibers 204, 304 and removal of the heat, the fibers 204, 304 may expand and contract, thereby moving the two structures relative to each other.

In various embodiments, the material used for fabricating the 3D-shaped fibers 204, 304 may include a thermoplastic having a volume that may change in response to or under one or more certain stimuli. As non-limiting examples, the volume change (e.g., an increase in volume) may be in the range of a few percentages to about 10 - 20 %, e.g., between about 1% and about 20%, between about 1% and about 15%, between about 1% and about 10%, between about 1% and about 5%, between about 5% and about 20%, between about 5% and about 15%, between about 5% and about 10%, or between about 10% and about 20%. For thermo-activated fibers (i.e., fibers responding to or changing in response to a thermal stimulus), it is preferable that the chosen thermoplastics have a high coefficient of thermal expansion, for example, ethylene-vinyl acetate and high-density polyethylene. For electrical-activated fibers, it is preferable that the chosen thermoplastics have a volume change in response to or under an electrical field, for example, polyacrylonitrile. Similarly, thermoplastics with a response to lighting, vapour, or chemical may be fabricated into fibers to respond to different stimuli. As non-limiting examples, thermal-responsive material may include poly (ε-caprolactone) (PCL), photo(light)-responsive material may include liquid crystal network coated polyethylene terephthalate (PET), chemical (vapour)-responsive material may include PCL with nanofillers (e.g., carbon nanotubes), and materials suitable for electrical induced actuation may include polyurethane. Non-limiting examples of chemical and/or vapour stimuli may include, but not limited to, water, acetone and chloroform. It should be appreciated that some thermoplastics may be responsive to multiple stimuli, while some thermoplastics which may not have a response to one or more certain stimuli may be functionalised with one or more proper second materials, thereby, forming a composite that may be responsive to the one or more certain stimuli.

Various embodiments may also provide fiber sensors. The sensors may include, but not limited to, temperature sensors, distance and/or force sensors, and lateral pressure sensors. The fiber sensors may be straight fibers. FIGS. 4A and 4B show schematic cross-sectional views of fiber sensors 460a, 460b, according to various embodiments. The fibers 460a, 460b may include a core 462a, 462b and a shell 464a, 464b.

Within thermoelectric (TE) technology, the Seebeck effect states that charge carriers thermally diffuse in a TE material under a temperature difference, resulting in an output voltage proportional to the temperature difference multiplied by the Seebeck coefficient of the material. Thus, this effect may be utilized to monitor temperature variation according to the corresponding output voltage and may be adopted in thermocouple applications. For fiber-shaped temperature (or thermal) sensors, or temperature sensing fibers, and using fibers 460a, 460b as non-limiting examples, each of the cores 462a, 462b and the shells 464a, 464b may be of any suitable shapes, e.g., square, rectangle, circle, etc. The cores 462a, 462b may include all types of thermoelectric glass, e.g., (Te₈₅Se₁₅)₄₅As₃₀Cu₂₅. The shells 464a, 464b may include all types of thermoplastic polymer (including thermoplastic elastomer), e.g., SEBS (styrene-ethylene-butylene-styrene), PEI (polyethylenimine), PC (polycarbonate), etc. Further non-limiting examples of thermoplastic polymers for the shells 464a, 464b may include polymers that may be synthesised in-house. These may include polymers/functional polymers in which the molecular chain structure and/or chemical bonds may be specifically manipulated. Through these synthesis, the polymers' properties may be designed and tailored to requirements or applications. Examples of such in-house synthesised polymers may include supramolecular hydrogel (e.g., βCD - Ad - Fc gel) with self-healing, expansion-contraction, and shape-memory properties, and thermoplastic polyurethane (TPU) with fast self-healing performance under room temperature.

Distance and/or force sensors may be designed based on the following. Resistance is inversely proportional to the cross-sectional area; for example, a thick copper wire has a lower resistance than a thin copper wire. Also, for a given material, the resistance is proportional to the length; for example, a long copper wire has a higher resistance than a short copper wire. The resistance R and conductance G of a conductor of uniform cross-section, therefore, may be computed as R = ρL/A. where L is the length of the conductor, measured in meters (m), A is the cross-sectional area of the conductor measured in square meters (m²), and ρ is the electrical resistivity (also called specific electrical resistance) of the material, measured in ohm-meters (Ω·m). Further, in linear elastomer, Young's modulus, Y, is known. Based on the relationship, F/A=Y ΔL/L, where A is the cross sectional area, F/A is the stress, ΔL is the length change of the fiber, L is the original length of the fiber, and ΔL/L is the strain, the force, F, may be determined based on F= AY ΔL/L.

For distance and/or force sensing fibers, and using fibers 460a, 460b as non-limiting examples, each of the cores 462a, 462b and the shells 464a, 464b may be of any suitable shapes, e.g., square, rectangle, circle, etc. The cores 462a, 462b may include all types of conductive (electrode) materials, including, e.g., conductive elastomer mixture of thermoplastic elastomer and conductive carbon powder, metal powder, etc. The shells 464a, 464b may include all types of thermoplastic polymer (including thermoplastic elastomer), e.g., SEBS (styrene-ethylene-butylene-styrene), PEI (polyethylenimine), PC (polycarbonate), in-house synthesised polymers as described above, etc.

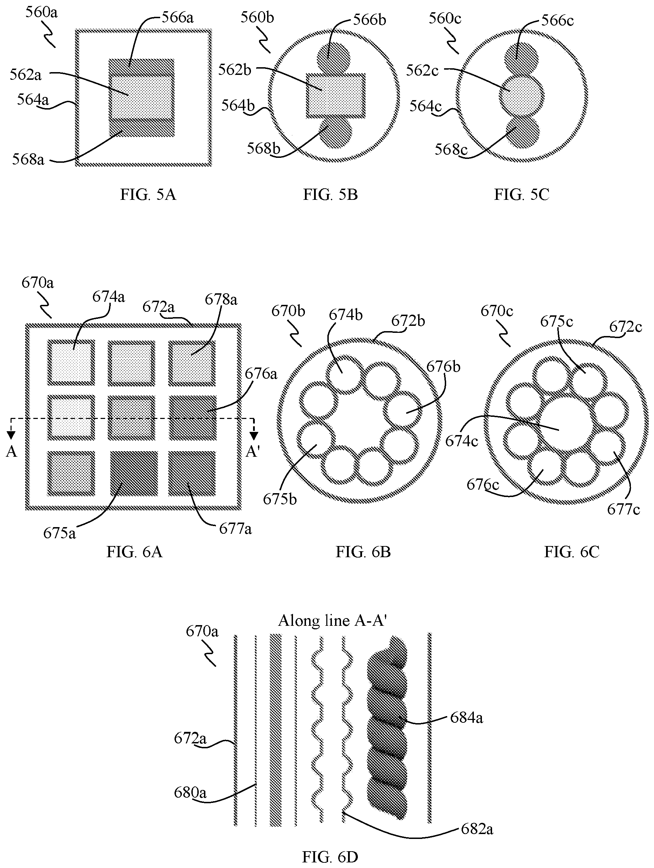

Lateral pressure sensors may be achieved based on electrostatic effect/capacitance change. FIGS. 5A to 5C show schematic cross-sectional views of lateral pressure sensing fibers 560a, 560b, 560c. The fibers 560a, 560b, 560c may include a core or medium region (or medium space) 562a, 562b, 562c, a shell 564a, 564b, 564c, and a pair of electrodes 566a, 568a, 566b, 568b, 566c, 568c. The electrodes 566a, 568a, 566b, 568b, 566c, 568c may be arranged on opposite sides of the medium region 562a, 562b, 562c. Each of the medium regions 562a, 562b, 562c, shells 564a, 564b, 564c, and electrodes 566a, 568a, 566b, 568b, 566c, 568c may be of any suitable shapes, e.g., square, rectangle, circle, etc. The medium regions 562a, 562b, 562c may act as a dielectric medium in a plane-parallel capacitor model defined together with the electrodes 566a, 568a, 566b, 568b, 566c, 568c. The medium regions 562a, 562b, 562c may include an (electrically) insulating material. The medium regions 562a, 562b, 562c may be deformable or flexible or elastic. The medium regions 562a, 562b, 562c may include air or liquid or polymer. The shells 564a, 564b, 564c may include all types of thermoplastic polymer (including thermoplastic elastomer), e.g., SEBS (styrene-ethylene-butylene-styrene), PEI (polyethylenimine), PC (polycarbonate), in-house synthesised polymers as described above, etc. The electrodes 566a, 568a, 566b, 568b, 566c, 568c may include a conductive material, including, e.g., metal, conductive polymer, etc.

When lateral pressure is applied on or to the fiber 560a, 560b, 560c, e.g., human hand touch and press, the electrostatic charges change on the surface of the shell 564a, 564b, 564c, and there may be charge transfer in the electrode components or electrodes 566a, 568a, 566b, 568b, 566c, 568c. The two electrodes 566a, 568a, 566b, 568b, 566c, 568c may be regarded as a plane-parallel capacitor. If the pressure applied changes the distance between two electrodes 566a, 568a, 566b, 568b, 566c, 568c, the capacitance of the capacitor (with two parallel plates) changes according to C = εS/4πkd (ε = relative dielectric constant, S = overlapping area of the two plates, k = Cullen constant/electrostatic force constant, d = distance between the two plates), where there may also be charge transfer in the electrodes 566a, 568a, 566b, 568b, 566c, 568c. Thus, lateral pressure may be sensed and collected. Therefore, it should be appreciated that charge transfer may occur not only under touch and slight press condition (e.g., where the distance between two electrodes may not or does not change), but also under a deep press condition (e.g., where the distance between two electrodes changes).

The fiber sensors of various embodiments, including the fiber sensors 460a, 460b, 560a, 560b, 560c, may be fabricated in a number of ways. One or more feasible methods may be chosen depending on the specific material property. Non-limiting examples of the fabrication method are described below.

One fabrication method involves multi-material thermal drawing in which a shell material, and, depending on the fiber design, one or more core materials, a medium region material, and one or more electrode materials, may be consolidated into a preform, which may then be drawn into a thin fiber together. In this condition, the design, with the relevant materials (e.g., cores), are already formed in the preform.

Another fabrication method may involve a wire convergence system during thermal drawing in which one or more functional wire materials are fed to respective pre-designed holes in the preform during the thermal drawing process, thereby forming a functional fiber when drawn. Depending on the fiber design, the wire material(s) may define one or more of core material(s), medium region material, and electrode material(s).

A further fabrication method may involve drawing a preform with one or more designed hollow cores into a fiber with one or more designed hollow cores first, and then injecting one or more functional materials into the respective holes in the fiber in a post-fabrication process. Depending on the fiber design, the functional material(s) may define one or more of core material(s), medium region material, and electrode material(s).

In various embodiments, the fiber-shaped structures described in the context of FIGS. 4A, 4B, and 5A to 5C may also be developed into an energy harvesting fiber, e.g., an energy harvesting fiber-based triboelectric nanogenerator which may also sense lateral pressure. Using FIGS. 5A to 5C as non-limiting examples, for the energy harvesting fibers, the medium regions 562a, 562b, 562c may be of any suitable shapes, e.g., square, rectangle, circle, etc., and may include air or liquid or polymer. The medium regions 562a, 562b, 562c are optional and the fiber-based triboelectric nanogenerator works with or without the medium regions 562a, 562b, 562c. The electrodes 566a, 568a, 566b, 568b, 566c, 568c may include conductive material, metal, liquid metal, conductive polymer, etc. The shells 564a, 564b, 564c may include a thermoplastic polymer that is compatible or matches with the electrode material.

A triboelectric nanogenerator converts mechanical energy into electric power with a combination effect of the electrification effect/triboelectric effect and electrostatic induction. Triboelectric effect is the phenomenon that a material becomes electrically charged after it contacts with a different material through friction. There are several working modes for triboelectric nanogenerators: contact-separation or lateral-sliding mode, single-electrode mode, and freestanding triboelectric-layer mode. For all the above modes, fiber triboelectric nanogenerators may harvest energy from the environment when there is charge induction and transfer. The energy may either be used directly to power a device (e.g., LED), or be stored in one or more energy storage units (e.g., supercapacitor or battery) for later use.

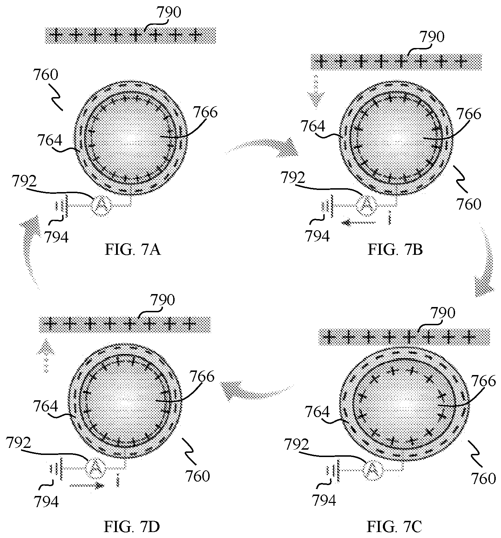

FIGS. 7A to 7D show schematic diagrams illustrating operation of an energy harvesting fiber (triboelectric nanogenerator) 760 of various embodiments, based on a single-electrode working mode as a non-limiting example. The harvesting fiber 760 may include a shell 764 surrounding an inner electrode 766. FIG. 7A shows the original charge distribution on or around the surface of the fiber 760 and the interface between the shell 764 and the inner electrode 766, which is in a static equilibrium state. For example, as may be observed, negative charges may be distributed on the shell 764 and positive charges may be distributed on the inner electrode 766 close to the interface. When an object 790 (e.g., an aluminium (Al) plate, a human hand, etc.) is brought close to the fiber 760 or into contact with the fiber 760, the charge distribution on the fiber 760 may be disturbed. The object 790 may have positive charges.

Referring to FIG. 7B, when the object 790 is moved from an original position illustrated in FIG. 7A towards the fiber 760, the distance between them decreases. When the fiber 760 and the object 790 are close enough, the positive charges on the object 790 may cause a decrease of positive charges in the electrode 766 through electrostatic induction effect. The positive charges on the inner electrode interface is reduced to keep the charge equilibrium, and this process is achieved by electrons flowing from the ground 794 towards the inner electrode 766 through an electrometer 792. When the object 790 contacts the fiber 760, as shown in FIG. 7C, a static equilibrium state is reached again. When the object 790 is moved away from the surface of the fiber 760, the positive charges on the surface of the electrode 766 increases, and to keep the charge equilibrium, electrons flow inversely from the electrode 766 to the ground 794, as shown in FIG. 7D. Then, the object 790 returns to the original state/position (see FIG. 7A) and a full cycle is completed.

Various embodiments may further provide one or more energy harvesting fibers having two electrodes, for example, based on the designs of FIGS. 5A to 5B, with or without a medium region. For an energy harvesting fiber with two same polarisation charged electrodes (i.e., both positive or both negative), the operation or process may correspond to two independent fibers assembled into one, where the operation of each fiber may be as described in the context of the fiber 760 of FIGS. 7A to 7D. For an energy harvesting fiber with two opposite charged electrodes (i.e., one positive and the other negative), a medium region (e.g., 562a, 562b, 562c of FIGS. 5A to 5C) may be provided to act as a dielectric medium between two plates of "plane-parallel capacitor" defined by the electrodes.

Fiber-based triboelectric nanogenerators and the operations thereof are further described on pages 241 to 257 in Advanced Fiber Sensing Technologies, Progress in Optical Science and Photonics Volume 9, 2020 (ISBN 978-981-15-5506-0; https://doi.org/10.1007/978-981-15-5507-7), the content of it being hereby incorporated by reference in its entirety for all purposes.

Each of the fiber sensors and the energy harvesting fiber of various embodiments may define a functional fiber.

Various embodiments may also provide fiber bundles, where each bundle may include at least one fiber actuator (e.g., accordion-like fiber 204, spring-like fiber 304) and at least one fiber sensor and/or energy harvesting fiber (e.g., functional fiber 460a, 460b, 560a, 560b, 560c). This may provide integration of fully functional fiber bundles for various applications.

The functional fiber(s) and fiber actuator(s) may, for example, be tied or warped together to form a fiber bundle, which becomes one thick fiber with, e.g., fully integrated sensing and actuation functions. For example, multiple hollow channels for filling in of sensing materials may be incorporated into fiber-shaped actuators for forming a self-sensing fiber actuation bundle for various applications such as artificial muscles. These hollow channels may be designed to locate fibers with different functions, , therby offering a way to bundle fibers.

As non-limiting examples, generally, a fiber actuator (or a functional fiber, e.g., fiber sensor) may be fabricated. A functional fiber (or a fiber actuator) with a hollow spacing in the fiber sensor (or the fiber actuator) may also be fabricated. There is no restriction as to forming the fiber actuator or the functional fiber first. Subsequently, the fiber actuator (or the functional fiber) may be fed or inserted into the hollow spacing. As a further non-limiting example, it may also be possible to form a fiber with a plurality of hollow channels, where a fiber actuator or a functional fiber may be inserted into a respective hollow channel. Further, it should be appreciated that the fabrication methods described above for fabricating the fiber sensors 460a, 460b, 560a, 560b, 560c, may also be applicable for forming the fiber bundles of various embodiments.

FIGS. 6A to 6C show schematic cross-sectional views illustrating integration of multifunctional fiber bundles 670a, 670b, 670c using fiber-shaped actuators and sensors/energy harvesters, according to various embodiments. Each fiber bundle 670a, 670b, 670c may include an outer shell 672a, 672b, 672c, of any suitable shapes, e.g., square, rectangle, circle, etc. Each fiber bundle 670a, 670b, 670c may include a plurality of functional regions (e.g., represented as 674a, 675a, 676a, 677a, 678a, 674b, 675b, 676b, 674c, 675c, 676c, 677c for some of the functional regions) of any suitable shapes, e.g., square, rectangle, circle, etc. Each functional region 674a, 675a, 676a, 677a, 678a, 674b, 675b, 676b, 674c, 675c, 676c, 677c may include a fiber actuator or a functional fiber (e.g., fiber sensor and/or energy harvesting fiber). Each functional region 674a, 675a, 676a, 677a, 678a, 674b, 675b, 676b, 674c, 675c, 676c, 677c may be a hollow channel initially which may then be filled with the relevant materials/fibers.

As a non-limiting example, the sensing materials filled into the hollow channels may include any material (e.g., polymer, metal, ceramic, etc) in any form (e.g., gas, liquid, solid, etc) as long as the filling material remains stable under the drawing temperature. The fiber shape, shape of hollow channels, and distribution of the channels may be adaptable as shown in FIGS. 6A to 6C.

As shown in FIGS. 6A to 6C, by filing the hollow channels with various materials/fibers, multiple functions, including sensing functions, may be integrated into one single fiber bundle 670a, 670b, 670c. The location for each fiber with a different function is not specifically fixed. There may be different permutations and combinations. As non-limiting examples using the fiber bundle 670a, the functional region 674a may include a thermal sensing fiber, the functional region 675a may include a muscle fiber (i.e., fiber actuator), the functional region 676a may include a muscle fiber (i.e., fiber actuator), the functional region 677a may include a pressure sensing fiber, and the functional region 678a may include an energy harvesting fiber. As a non-limiting example, FIG. 6D shows a schematic cross-sectional view along line A-A' shown in FIG. 6A where the fiber bundle 670a may include a fiber sensor 680a, an accordion-like fiber 682a, and a spring-like fiber 684a.

Artificial muscle system is a critical part of soft robotics to offer the strength for body motion and response to the environment based on sensing ability. Fiber-based artificial muscle system provides one or more of the following features, such as lightweight, large power-to-mass ratio, tunable size from micrometer-to-centimeter, low-cost scalable fabrication. As detecting temperature, distance, position, power, and pressure may be achieved through structure and material design for fiber sensors in thermal drawing technology, various embodiments may provide a strategy to fabricate a fully functional fiber artificial muscle system with integrated sensing-actuating and self-power supply functions, which may push the muscle system in soft robotics to move forward to the next milestone stage.

Various embodiments and the techniques disclosed herein may offer one or more of the following features, compared to the existing technologies: (i) multiple sensing functions may be integrated compared with reported fiber bundles, including distance, force, temperature, and pressure sensing, making it a fully functional fiber-based system, (ii) with various structures and material designs in one single fiber bundle, the applications of the fiber bundle may be extended, (iii) by adding energy harvesting fibers, a self-powered fiber sensing system may be achieved, and (iv) fexibility in materials selection, scalable production, and more wearable-friendly compared to known existing fiber bundles, especially for artificial muscles.

Various embodiments may offer one or more of the following (including commercial applications): (i) fully functional fiber artificial muscle system with sensing and monitoring performance, (ii) the use of widely available materials and scalable fabrication techniques, (iii) wearable power supply for wearable electronics or portable devices, (iv) accelerating the development of robotics, haptics, and prosthetics, and (v) extending the fiber fabrication technology impact from engineering fields to biomedical applications.

## Claims

1. A method (120) of forming a fiber comprising:
subjecting (122) a preform (200) to a thermal drawing process to draw the preform into a fiber (202); and
applying (124), at intervals, a compressive force to sections of the fiber (202) along a length of the fiber (202) during the thermal drawing process to reduce a dimension of the sections to form the fiber (202) into an accordion-like fiber (204).

2. The method as claimed in claim 1, wherein applying (124) the compressive force comprises:
passing the fiber (202) through an iris (208); and
closing the iris (208), at the intervals, to apply the compressive force to the sections of the fiber (202),
wherein, preferably, a thickness of the iris (208) is between about 0.05 mm and about 0.5 mm.

3. The method as claimed in claim 1 or 2,
wherein the thermal drawing process comprises heating the preform (200) at a drawing temperature of between about 100°C and about 500°C, and/or
wherein the thermal drawing process comprises drawing the preform (200) into the fiber (202) at a drawing rate of between about 0.1 m/min and about 10 m/min, and/or
wherein the compressive force applied to the sections of the fiber (202) is between about 0.5 N and about 10 N.

4. The method as claimed in any one of claims 1 to 3,
wherein applying the compressive force to the sections of the fiber (202) comprises applying the compressive force to reduce the sections to between about 30% and about 70% of a diameter of the fiber (202), and/or
wherein applying the compressive force to the sections of the fiber (202) comprises applying the compressive force at time intervals of between about 0.001s and about 3s, and/or
wherein applying the compressive force to the sections of the fiber (202) comprises applying, for each of the sections of the fiber, the compressive force for between about 0.001s and about 3s.

5. The method as claimed in any one of claims 1 to 4,
wherein a spacing between adjacent sections of the sections of the fiber (202) is between about 0.05 mm and about 10 mm, and/or
wherein the sections of the fiber (202) comprise between about 10% and about 80% of the fiber (202), and/or
wherein the preform (200) comprises a thermoplastic, wherein, preferably, the thermoplastic comprises a material that is responsive to at least one of heat, electric field, light, chemical or vapour.

6. A fiber bundle (170, 670a, 670b, 670c) comprising:
at least one fiber actuator (174), wherein the fiber actuator (174) comprises the accordion-like fiber (204) formed by the method as claimed in any one of claims 1 to 5; and
at least one functional fiber (176, 460a, 460b, 560a, 560b, 560c, 760) configured to function as at least one of a sensor or an energy harvester.

7. The fiber bundle as claimed in claim 6,
wherein the at least one functional fiber (460a, 460b) comprises a shell (464a, 464b) comprising a thermoplastic polymer, and/or
wherein the at least one functional fiber (460a, 460b) comprises a core (462a, 462b) comprising a thermoelectric glass or a conductive material, and/or
wherein the at least one functional fiber (560a, 560b, 560c) comprises a pair of electrodes (566a, 568a, 566b, 568b, 566c, 568c) arranged spaced apart, wherein, preferably, the at least one functional fiber further (560a, 560b, 560c) comprises an insulative core (562a, 562b, 562c), wherein the pair of electrodes (566a, 568a, 566b, 568b, 566c, 568c) are arranged on opposite sides of the insulative core (562a, 562b, 562c), wherein, preferably, the insulative core (562a, 562b, 562c) is deformable.

8. A method (140) of forming a fiber bundle (170, 670a, 670b, 670c) comprising:
arranging at least one fiber actuator (174), and at least one functional fiber (176, 460a, 460b, 560a, 560b, 560c, 760) configured to function as at least one of a sensor or an energy harvester to form the fiber bundle,
wherein, preferably, arranging the at least one fiber actuator (174) and the at least one functional fiber (176, 460a, 460b, 560a, 560b, 560c, 760) to form the fiber bundle (170, 670a, 670b, 670c) comprises:
forming the at least one fiber actuator (174) according to the method as claimed in any one of claims 1 to 5;
forming the at least one functional fiber (176, 460a, 460b, 560a, 560b, 560c, 760);
defining a hollow spacing in one of the at least one fiber actuator (174) and the at least one functional fiber (176, 460a, 460b, 560a, 560b, 560c, 760); and
inserting the other of the at least one fiber actuator (174) and the at least one functional fiber (176, 460a, 460b, 560a, 560b, 560c, 760) into the hollow spacing.

## Patentansprüche

1. Verfahren (120) zum Bilden einer Faser, umfassend:
Unterziehen (122) einer Vorform (200) einem thermischen Ziehverfahren, um die Vorform in eine Faser (202) zu ziehen; und
Anwenden (124) einer Druckkraft auf Abschnitte der Faser (202) entlang einer Länge der Faser (202) während des thermischen Ziehprozesses, um eine Dimension der Abschnitte zu reduzieren, um die Faser (202) zu einer akkordeonartigen Faser (204) zu formen.

2. Verfahren nach Anspruch 1, wobei das Aufbringen (124) der Druckkraft umfasst:
Führen der Faser (202) durch eine Iris (208); und
Schließen der Iris (208) in den Intervallen, um die Druckkraft auf die Abschnitte der Faser (202) aufzubringen,
wobei vorzugsweise eine Dicke der Iris (208) zwischen etwa 0,05 mm und etwa 0,5 mm liegt.

3. Verfahren nach Anspruch 1 oder 2,
wobei der thermische Ziehprozess das Erhitzen der Vorform (200) auf eine Ziehtemperatur zwischen etwa 100°C und etwa 500°C umfasst, und/oder
wobei das thermische Ziehverfahren das Ziehen der Vorform (200) in die Faser (202) mit einer Ziehgeschwindigkeit zwischen etwa 0,1 m/min und etwa 10 m/min umfasst, und/oder
wobei die Druckkraft, die auf die Abschnitte der Faser (202) ausgeübt wird, zwischen etwa 0,5 N und etwa 10 N liegt.

4. Verfahren nach einem der Ansprüche 1 bis 3,
wobei das Aufbringen der Druckkraft auf die Abschnitte der Faser (202) das Aufbringen der Druckkraft umfasst, um die Abschnitte auf etwa 30 % bis etwa 70 % eines Durchmessers der Faser (202) zu reduzieren, und/oder
wobei das Aufbringen der Druckkraft auf die Abschnitte der Faser (202) das Aufbringen der Druckkraft in Zeitintervallen zwischen etwa 0,001 s und etwa 3 s umfasst, und/oder
wobei das Aufbringen der Druckkraft auf die Abschnitte der Faser (202) das Aufbringen der Druckkraft für jeden der Abschnitte der Faser für etwa 0,001 s bis etwa 3 s umfasst.

5. Verfahren nach einem der Ansprüche 1 bis 4,
wobei ein Abstand zwischen benachbarten Abschnitten der Abschnitte der Faser (202) zwischen etwa 0,05 mm und etwa 10 mm liegt, und/oder
wobei die Abschnitte der Faser (202) zwischen etwa 10 % und etwa 80 % der Faser (202) umfassen, und/oder
wobei die Vorform (200) einen Thermoplast umfasst, wobei der Thermoplast vorzugsweise ein Material umfasst, das auf mindestens eines von Wärme, elektrischem Feld, Licht, Chemikalie oder Dampf reagiert.

6. Faserbündel (170, 670a, 670b, 670c) umfassend:
mindestens einen Faseraktuator (174), wobei der Faseraktuator (174) die akkordeonartige Faser (204) umfasst, die nach dem Verfahren nach einem der Ansprüche 1 bis 5 gebildet wird; und
mindestens eine funktionsfähige Faser (176, 460A, 460B, 560A, 560B, 560C, 760), die so konfiguriert sind, dass sie mindestens als ein Sensor oder ein Energiegewinnungsgerät (Energy Harvester) fungiert.

7. Faserbündel nach Anspruch 6,
wobei die mindestens eine Funktionsfaser (460a, 460b) eine Hülle (464a, 464b) umfasst, die ein thermoplastisches Polymer umfasst, und/oder
wobei die mindestens eine Funktionsfaser (460a, 460b) einen Kern (462a, 462b), aus einem thermoelektrischen Glas oder einem leitfähigen Material umfasst, und/oder
wobei die mindestens eine Funktionsfaser (560a, 560b, 560c) ein Paar von Elektroden (566a, 568a, 566b, 568b, 566c, 568c) umfasst, die voneinander beabstandet angeordnet sind, wobei vorzugsweise die mindestens eine Funktionsfaser ferner (560a, 560b, 560c) einen isolierenden Kern (562a, 562b, 562c) umfasst, wobei das Elektrodenpaar (566a, 568a, 566b, 568b, 566c, 568c) auf gegenüberliegenden Seiten des isolierenden Kerns (562a, 562a, 562b, 562c) angeordnet ist, wobei vorzugsweise der Isolationskern (562a, 562b, 562c) verformbar ist.

8. Verfahren (140) zum Bilden eines Faserbündels (170, 670a, 670b, 670c), umfassend:
Anordnung von mindestens einem Faseraktuator (174) und mindestens einer Funktionsfaser (176, 460A, 460B, 560A, 560B, 560C, 760), die so konfiguriert ist, dass sie mindestens als ein Sensors oder ein Energiegewinnungsgerät (Energy Harvester) fungiert, um das Faserbündel zu bilden,
wobei vorzugsweise die Anordnung des mindestens einen Faseraktuators (174) und der mindestens einen Funktionsfaser (176, 460a, 460b, 560a, 560b, 560c, 760) zur Bildung des Faserbündels (170, 670a, 670b, 670c) umfasst:
Bilden des mindestens einen Faseraktuators (174) gemäß dem Verfahren, wie es in einem der Ansprüche 1 bis 5 beansprucht wird;
das Bilden der mindestens einen funktionellen Faser (176, 460A, 460B, 560A, 560B, 560C, 760);
Definieren eines Hohlraums in einem dem mindestens einen Faseraktuator (174) und der mindestens einen Funktionsfaser (176, 460a, 460b, 560a, 560b, 560c, 760); und
Einsetzen der anderen der mindestens einen Faseraktuatoren (174) und der mindestens einen Funktionsfaser (176, 460A, 460B, 560A, 560B, 560C, 760) in dem Hohlraum.

## Revendications

1. Procédé (120) de formation d'une fibre comprenant :
soumettre (122) une préforme (200) à un procédé d'emboutissage thermique pour aspirer la préforme en une fibre (202) ; et
en appliquant (124), à intervalles réguliers, une force de compression à des sections de la fibre (202) le long d'une longueur de la fibre (202) pendant le processus d'emboutissage thermique pour réduire une dimension des sections afin de former la fibre (202) en une fibre (204) en accordéon.

2. Procédé selon la revendication 1, dans lequel l'application (124) de la force de compression comprend :
passage de la fibre (202) à travers un iris (208) ; et
fermer l'iris (208), aux intervalles, pour appliquer la force de compression sur les sections de la fibre (202),
dans lequel, de préférence, l'épaisseur de l'iris (208) est comprise entre 0,05 mm et 0,5 mm environ.

3. Procédé selon la revendication 1 ou 2,
dans lequel le procédé d'emboutissage thermique comprend le chauffage de la préforme (200) à une température d'emboutissage comprise entre environ 100°C et environ 500°C, et/ou
dans lequel le procédé d'étirage thermique comprend l'étirage de la préforme (200) dans la fibre (202) à une vitesse d'étirage comprise entre environ 0,1 m/min et environ 10 m/min, et/ou
où la force de compression appliquée aux sections de la fibre (202) est comprise entre environ 0,5 N et environ 10 N.

4. Procédé selon l'une quelconque des revendications 1 à 3,
dans lequel l'application de la force de compression aux sections de la fibre (202) comprend l'application de la force de compression pour réduire les sections entre environ 30 % et environ 70 % d'un diamètre de la fibre (202), et/ou
dans lequel l'application de la force de compression sur les sections de la fibre (202) comprend l'application de la force de compression à des intervalles de temps compris entre environ 0,001 s et environ 3 s, et/ou
dans lequel l'application de la force de compression sur les sections de la fibre (202) comprend l'application, pour chacune des sections de la fibre, de la force de compression pendant environ 0,001s à environ 3s.

5. Procédé selon l'une quelconque des revendications 1 à 4,
dans lequel un espacement entre les sections adjacentes des sections de la fibre (202) est compris entre environ 0,05 mm et environ 10 mm, et/ou
dans laquelle les sections de la fibre (202) comprennent entre environ 10 % et environ 80 % de la fibre (202), et/ou
dans lequel la préforme (200) comprend un thermoplastique, dans lequel, de préférence, le thermoplastique comprend un matériau qui réagit à au moins un de la chaleur, du champ électrique, de la lumière, de l'un chimique ou de la vapeur.

6. Un faisceau de fibres (170, 670a, 670b, 670c) comprenant :
au moins un actionneur de fibre (174), dans lequel l'actionneur de fibre (174) comprend la fibre en accordéon (204) formée par le procédé selon l'une quelconque des revendications 1 à 5 ; et
au moins une fibre fonctionnelle (176, 460a, 460b, 560a, 560b, 560c, 760) configuré pour fonctionner comme au moins l'un des capteurs ou des récupérateurs d'énergie.

7. Le faisceau de fibres selon la revendication 6,
dans lequel la ou les fibres fonctionnelles (460a, 460b) comprennent une enveloppe (464a, 464b) comprenant un polymère thermoplastique, et/ou
dans laquelle la ou les fibres fonctionnelles (460a, 460b) comprennent un noyau (462a, 462b) comprenant un verre thermoélectrique ou un matériau conducteur, et/ou
dans lequel la ou les fibres fonctionnelles (560a, 560b, 560c) comprennent une paire d'électrodes (566a, 568a, 566b, 568b, 566c, 568c) disposées espacées, dans laquelle, de préférence, la ou les fibres fonctionnelles (560a, 560b, 560c) comprennent un noyau isolant (562a, 562b, 562c), dans lequel la paire d'électrodes (566a, 568a, 566b, 568b, 566c, 568c) sont disposées sur les côtés opposés du noyau isolant (562a, 562b, 562c), dans lequel, de préférence, le noyau isolant (562a, 562b, 562c) est déformable.

8. Procédé (140) de formation d'un faisceau de fibres (170, 670a, 670b, 670c) comprenant :
disposant au moins un actionneur de fibre (174) et au moins une fibre fonctionnelle (176, 460a, 460b, 560a, 560b, 560c, 760) configuré pour fonctionner comme au moins l'un des capteurs ou d'un récupérateur d'énergie pour former le faisceau de fibres,
dans lequel, de préférence, l'agencement du ou des actionneurs de fibre (174) et de la ou des fibres fonctionnelles (176, 460a, 460b, 560a, 560b, 560c, 760) pour former le faisceau de fibres (170, 670a, 670b, 670c) comprend :
formant le ou les actionneurs à fibre (174) selon le procédé selon l'une quelconque des revendications 1 à 5 ;
formant la ou les fibres fonctionnelles (176, 460A, 460B, 560A, 560B, 560C, 760) ;
définition d'un espacement creux dans l'un des au moins un actionneur de fibre (174) et dans la ou les fibres fonctionnelles (176, 460a, 460b, 560a, 560b, 560c, 760) ; et
en insérant l'autre de l'au moins un actionneur de fibre (174) et de la ou des fibres fonctionnelles (176, 460A, 460B, 560A, 560B, 560C, 760) dans l'espacement creux.
